# EUROPEAN PATENT APPLICATION

(11) **EP 4 555 972 A1**
(43) Date of publication of application: **21.05.2025**
(21) Application number: 23838348.3
(22) Date of filing: 14.07.2023
(51) Int. Cl.: A61C 13/01, A61C 8/00, C25D 11/02, A61L 27/06

(54) **PROCESS FOR MANUFACTURING A DENTAL IMPLANT, DEVICE FOR ANODISATION OF A DENTAL IMPLANT, AND DEVICE FOR ACID ETCHING AND COATING OF A DENTAL IMPLANT**

(30) Priority: 15.07.2022 BR 102022014113
(71) Applicant: M3 Health Indústria e Comércio de Produtos Médicos, Odontológicos e Correlatos S.A., CEP: 13212-213 Jundiaí (BR)
(72) Inventor: BLAY, Alberto, CEP 01250-040 São Paulo (BR); DE PALMA TEGON, Andre, CEP: 13251-500 Itatiba (BR); PEREIRA DA SILVA, Daniel, CEP: 07417-450 Arujá (BR); SASKA JUNIOR, Luis Antonio, CEP: 14806-153 Araraquara (BR)
(74) Representative: Pereira Garcia, João Luís
(86) International application number: PCT/BR2023/050236
(87) International publication number: WO 2024/011304

(57) **Abstract**

The present invention describes a 3D printed dental implant manufacturing process in which a chemical treatment process is carried out, preferably acid etching, on the external surface of said implant; and an anodizing process on the inner surface of the cavity of said implant.

Furthermore, the present invention refers to a device for aligning an implant body for the CNC machining process through a clamp holder shaft; an implant cavity anodizing device; and a device for etching the outer surface of an implant configured to be implemented in an implant manufacturing process, wherein the implant manufacturing process can be automated and performed on multiple implants simultaneously.

## Description

### FIELD OF INVENTION

. The present invention belongs to the technological sector of dentistry and refers to a process for anodizing dental implants using a device.

. Still, the present invention refers to the process of machining the implant in a machining machine.

. Furthermore, the present invention refers to a device for anodizing the internal part of dental implants.

. Furthermore, the present invention refers to a device for chemical treatment and coating of the surface of the external region of dental implants.

### BACKGROUND OF THE INVENTION

. In the fields of medical devices related to implant dentistry, it is possible to observe the growing demand for solutions to problems of acceptability of the patient's body to implants made of synthetic material, through the optimization of osseointegration and the reduction of accumulation of bacteria on the surface of the implant and its interfaces.

. Anodizing is a chemical process, carried out in baths based on alkaline and acidic substances, with an electric current that forms a protection of titanium oxide. As it is formed from this electrochemical reaction of titanium, the layer is integrated into the profile.

. In this sense, some solutions and technologies have already been developed to solve these inconveniences.

. Document BR1120180163287 discloses a process for treating an implant, comprising a step of treating the surface of the implant with at least one phosphonic acid compound. In machined implants, there is no concern that acid attack will compromise the external structures of the implant. However, additive manufacturing makes it possible for impossible-to-machine shapes and structures to be formed on the implant surface during fabrication. Submitting these implants manufactured by addition to surface treatment, anodizing and acid etching processes would compromise the external structures purposely formed in the additive manufacturing process.

. Document CN213552516 discloses a dental implant surface acid corrosion treatment device comprising a constant temperature water tank and an etching tool, the etching tool comprising a tool cover plate, a tool base, a clamp spacer column, a positioning spacer column and a spacer column, the lower part of the tool cover plate is connected to the tool base through the positioning spacer column, and the tool clamp is connected to the tool base through the spacer column.

**.** Document CN212630936 discloses an acid treatment device applied to the surface of a dental implant, comprising a rack, a liquid storage tank and a lifting mechanism, which are arranged on the shelf, the lifting mechanism is fixedly connected with a support rod, the other end of the support rod is connected to the center of a dental implant fixation disc, and a drive motor is disposed between the center of the dental implant fixation disc and the support rod.

**.** Document CN107447216 describes a surface treatment device for the dental implant being an oxidation tool with automatic centering, therefore, the difficulty of positioning the oxidation tool can be reduced, the displacement of the oxidation tool in the process of moving between stations can be avoided.

**.** With the emergence of new surface treatment techniques, anodizing the external part of an implant, particularly an implant obtained through additive manufacturing, has an undesirable effect on the final result of the implant, since it would compromise the geometries of micro and nanostructures of the external surface, inherent in the additive manufacturing process, and compromising the efficiency of this surface in the osseointegration process; however, the identification by color of the different prosthetic platforms helps in the adequate choice of prosthetic components in relation to the diameter of the platform (example, dental implants internally anodized in pink, only prosthetic components of the same line as the implants in pink color can be connected to this platform).

**.** As can be seen, in the state of the art there is no solution that allows the anodizing of the internal part, independently of the external part, of multiple dental implants simultaneously, which is a laborious process.

**.** Additionally, it is important to preserve the internal part of the implants both before and after anodizing in acid etching processes and coating of its external surface.

**.** To solve these and other problems, the present invention conceives a process for manufacturing a dental implant from an implant body joined to a manufactured implant connection and alignment element, preferably by additive manufacturing, in which the additional part, clamp holder shaft, is joined in order to conceive a high precision alignment of the implant to the CNC machining machine.

**.** In another aspect of the present invention, the present invention conceives a device for accommodating multiple dental implants that receives said implants and performs a sealed closure in layers, where the internal surface of the implant is isolated from the external surface, allowing access and the realization of acid attack from the inside, without the acid coming into contact with the outside. The device makes it possible to systematize the process of anodizing internal surfaces for multiple implants at once, simplifying its manufacture, and automating and increasing the production of implants with an internal anodized surface, making its manufacturing process cheaper, with reduced use of solutions and waste during the manufacturing process compared to other conventional methods.

**.** In another aspect of the present invention, the present invention conceives a device for accommodating multiple dental implants that receives said implants by fixing the internal thread of the implant, so that its interior is isolated, and only the external surface of the implants is exposed for treatments intended only for the external surface, preserving the internal structural properties of the implant.

**.** Therefore, there is no state-of-the-art solution equivalent to the one presented here in the present invention that combines technical differentials, economic advantages, reliability and safety.

### OBJECTIVES OF THE INVENTION

**.** Thus, it is an objective of the present invention to provide a solution to the challenges and limitations listed above, presenting a manufacturing process for a dental implant with different surface treatments in its internal and external region.

**.** Furthermore, it is an objective of the present invention to provide a device for accommodating multiple dental implants that receives said implants and performs a sealed closure in layers, where the internal surface of the implant is isolated from the external surface.

**.** Furthermore, it is an objective of the present invention to provide a device that allows access to the internal surface of the implants, in order to allow substances for chemical processes to come into contact only with the internal surface of the implants.

**.** Furthermore, it is an objective of the present invention to provide a device that allows access to the external surface of the implants, in order to allow substances for chemical processes to come into contact only with the external surface of the implants.

**.** It is also an object of the present invention to provide a reliable and secure seal between the inside and outside of the implant while the implant is contained in the device.

**.** It is also an objective of the present invention to provide a device that allows the process of anodizing the internal surface of multiple implants simultaneously.

**.** It is also an objective of the present invention to provide a device that allows the process of chemical treatment of the external surface of multiple implants simultaneously.

**.** It is also an objective of the present invention to provide a process that preserves the characteristics of the external surfaces and the part of the internal prosthetic connection obtained in previous manufacturing steps.

### SUMMARY OF THE INVENTION

**.** In one aspect of the present invention, these and other objectives are achieved through the implant manufacturing process comprising:
- a 3D printed dental implant;
- a clamp holder shaft made of metallic material fused to the end of the implant body, in order to form a single piece;
- the clamp holder shaft further comprising an elongated region configured to be fixed to a guide bush;
the clamp holder shaft also comprising a stop that delimits the depth of the elongated region that will enter the guide bush and the guide bush keeps said shaft attached to a turning machine, and the stop rests against the guide bush, so that the relative distance from the shaft for guide bush, measured in the direction of the shaft of rotation of the turning machine, be a constant distance.

**.** In another aspect of the present invention, these and other objectives are achieved through a machining process guided by a clamp holder shaft comprising the following steps:
- manufacture of a clamp holder shaft next to the dental implant by means of selective laser fusion (single body piece);
- fixation of the single body to a guide bush by means of the clamp holder shaft;
- attachment of the single body to a turning machine (CNC) through the guide bush;
- shear between the implant body and the clamp holder shaft;
- machining of the prosthetic connection of the internal surface of the implant body in order to obtain a 3D printed dental implant with the internal part machined.

**.** In another aspect of the present invention, these and other objectives are achieved by means of a device for anodizing a dental implant comprising:
- a base plate;
- said base plate comprising a set of guide pins configured to engage in the internal part of dental implants;
   - a housing plate;
- said housing plate comprising a set of holes configured so as to align with the guide pins of the base plate, said holes traversing the entire housing plate;
- a sealing structure;
- said sealing structure being configured to match the position of the set of guide pins and the position of the holes;
- a bottom plate;
- a plurality of guide pins, configured to align the base, housing and top plates; and
- locking elements, configured in such a way as to fix and lock the housing plate next to the bottom plate;
wherein the base has fitting means and gripping means and the housing plate further comprises fitting means, a recessed region with ramp, and fastening means, and the bottom plate comprises fitting means configured to match the fitting means of the housing plate and fastening means configured to match the fastening means of the housing plate.

**.** In another aspect of the present invention, these and other objectives are achieved through a dental implant anodizing process comprising the following steps:
- assembly of the device with implant comprising:
   - position the base so that the guide pins face upwards;
   - attach the housing plate, so that the guide pins pass through the holes in the housing plate;
   - insert the height guides by fitting the base and the housing plate;
   - place the sealing structure on the housing plate so that it is around the tip of the guide pins;
   - insert dental implants into the device, so that the internal part of the implant fits over the tip of the guide pins, and its coronal end is in contact with the sealing structure;
   - couple the bottom plate under the device and aligning said bottom plate by engaging the fitting means with the height guides;
   - insert locking elements through the fastening means; and
   - lock the locking elements in order to join the housing plate to the bottom plate;
   - removal of the base together with the guide pins;
   - pour an anodizing solution into the recessed region of the housing plate so that said solution enters the holes and comes into contact with the internal region of the implant;
   - remove the locking elements and separate the bottom plate and housing plate; and
   - remove the anodized implants.

**.** In another aspect of the present invention, these and other objectives are achieved by means of a device for chemical treatment and coating of a dental implant comprising:
- a sealing shaft;
- the sealing shaft comprising a through hole;
- the through hole being surrounded by sealing nozzles at each end of the hole;
- an implant fixation pin;
- the implant fixation pin comprising two thread regions;
wherein said through hole is configured to receive said fixation pin and the two thread regions are configured to receive the internal thread of a dental implant, so that when passing said fixation pin by said sealing shaft, at least two implant fixation regions are created, so that the coronal region of the implant is in contact with the nozzles and access to the internal surface of the implant it is impossible.

**.** In another aspect of the present invention, these and other objectives are achieved through a process for chemical treatment comprising the following steps:
- assembly of the chemical treatment device comprising:
   - passage of the implant fixation pin through the sealing shaft, so that each end of the chemical treatment device has a thread region;
   - thread of the internal thread of the implants in both thread regions, so that the coronal ends of the implants make contact with the sealing nozzles, preventing a liquid solution from coming into contact with the internal part of the implants;
- immersion of the device with implant in a treatment solution for a sufficient time;
- removal the device from the treatment solution;
- connection and immersion of the device (31) in a solution containing carbon nanostructures (graphene oxide, reduced graphene oxide, graphene, carbon nanotubes etc.);
- application of electric current for a determined time according to the number of implants and the type of coverage desired; and
- removal of the implants.

**.** In another aspect of the present invention, these and other objectives are achieved through a manufacturing process for a dental implant, bringing together steps from the different processes mentioned herein.

### BRIEF DESCRIPTION OF THE DRAWINGS

**.** The present invention will be described based on the attached drawings, which illustrate:
Figure 1 illustrates the front view of the single body formed by a clamp holder shaft attached to an additive manufacturing implant body, object of the present invention in a preferred embodiment.
Figure 2 illustrates the perspective view of the single body assembled joined to an additive manufacturing implant, object of the present invention in a preferred embodiment.
Figure 3 illustrates the exploded view of the anodizing device, object of the present invention in a preferred embodiment.
Figure 4 illustrates the perspective view of the anodizing device, object of the present invention completely assembled and closed, in a preferred embodiment.
Figure 5 illustrates the assembly step of the anodizing device in which the base is coupled to the housing plate.
Figure 6 illustrates the assembly stage of the anodizing device in which the height guides and the sealing structure are inserted.
Figure 7 illustrates the perspective view of the assembled structure of the anodizing device to receive the implants.
Figure 8 illustrates the assembly step of the anodizing device in which the dental implants are inserted into the tip of the base guide pins under the sealing structure.
Figure 9 illustrates the assembly stage of the anodizing device in which the bottom plate is coupled.
Figure 10 illustrates the assembly stage of the anodizing device in which the locking elements are coupled.
Figure 11 illustrates a perspective view of the anodizing device, object of the present invention, completely assembled and ready to receive the treatment solution, after removing the base plate, in a preferred embodiment.
Figure 12 illustrates the top view of the anodizing device, object of the present invention, completely assembled and ready to receive the treatment solution, after removing the base plate, in a preferred embodiment.
Figure 13 illustrates the exploded front view of the chemical treatment device for receiving the implants in a preferred embodiment.
Figure 14 illustrates the perspective view of the chemical treatment device assembled to receive the implants in a preferred embodiment.
Figure 15 illustrates the front view of the chemical treatment and coating device with the fixed implants, in a preferred embodiment.

### DETAILED DESCRIPTION OF THE INVENTION

**.** The present invention relates to a manufacturing process for an implant in three main steps: machining of the internal prosthetic connection of the 3D printed dental implant; anodization of the internal part of dental implants and the chemical treatment of the external part of the implant. The present invention uses a clamp holder shaft for the alignment of 3D printed dental implants in the machine for machining the internal part of said implants to form the prosthetic connection, an internal anodizing device and an external treatment device with innovative constructions that facilitate each step, and standardize them in a way that facilitates automation and/or that a plurality of implants is submitted to each step in each cycle.

**.** A clamp holder shaft is designed using an innovative construction with a protrusion that facilitates alignment on CNC lathe machines.

**.** A device is also conceived that uses overlapping plates, to segment the sectors of interest of the implant, sealing the internal and external surfaces, while still allowing access to the internal surface so that it receives the necessary treatment solution.

**.** A device is also conceived that uses a sealing body, to segment the sectors of interest of the implant, sealing the internal and external surfaces, while still allowing access to the external surface so that it receives the necessary treatment solution.

**.** It should be noted that, as already demonstrated, the state of the art does not offer a solution equivalent to the invention described here. Thus, the present invention presents numerous technical and economic advantages when compared to the state of the art, some of which are listed below:
- The present invention constitutes an innovative process through devices that allow the complete isolation of the internal and external surface of the implant, allowing each surface to receive a different surface treatment, which best suits the needs of each part of the implant;
- The present invention allows implants manufactured by additive manufacturing methods to maintain the inherent characteristics and structures of the method on its external surface, allowing only the internal region of the implant to be anodized;
- The present invention preserves the anodized internal region of the implant in chemical treatment processes on the external surface and coating;
- The present invention presents an efficient process, compatible with modern manufacturing processes, facilitating its automation;
- The present invention simplifies the internal surface treatment process for multiple implants and simultaneously;

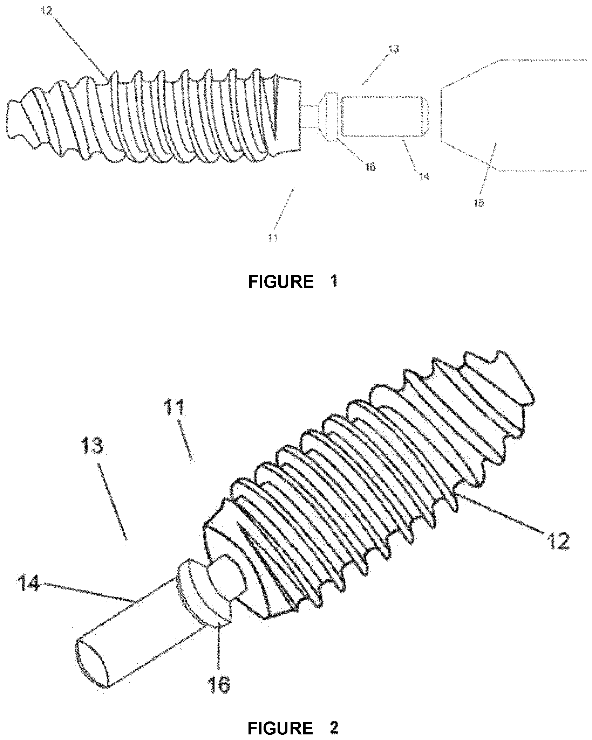

. With reference to Figure 1, the present invention comprises a single body 11. In a preferred embodiment of the present invention, the single body 11 comprises an implant body 12 and a clamp holder shaft 13.

. In a preferred form of the invention, the clamp holder shaft 13 is made of metallic material and is manufactured via additive manufacturing (selective laser melting) at the end of the implant body 12, so as to form a single piece.

. The clamp holder shaft 13 comprises an elongated region 14 configured to be attached to a guide bush 15, which keeps said single body 11 attached to a turning machine. The clamp holder shaft 13 also comprises a stop 16 that delimits the depth of the elongated region 14 that will enter the guide bush 15 of the clamp of the turning machine.

**.** Preferably, the single body 11 can be made of at least one material among: titanium alloys, nickel alloys, chromium alloys, aluminum alloys and stainless steel.

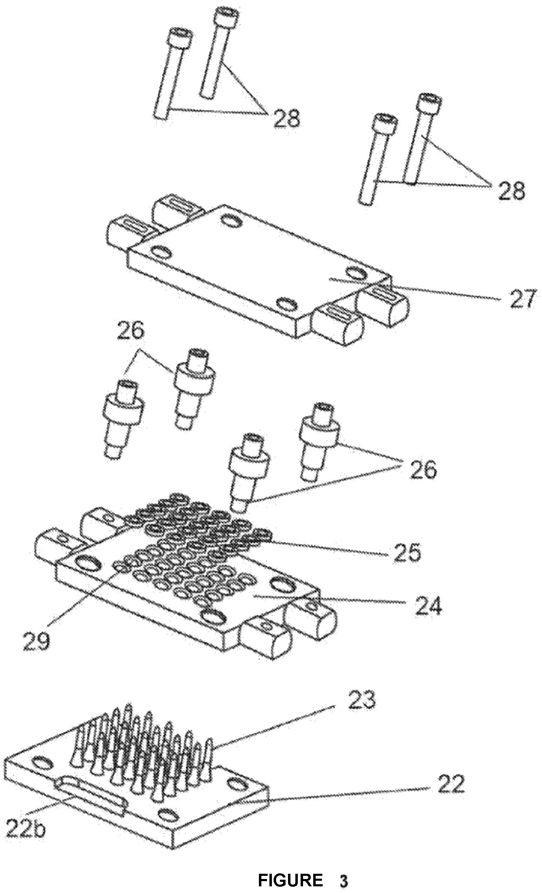

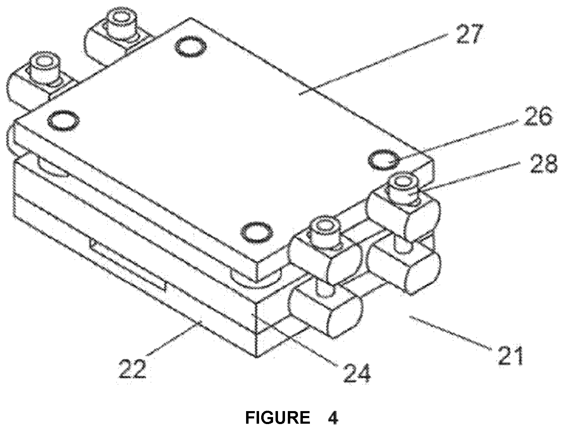

**.** With reference to Figures 3 to 4, the present invention comprises an anodizing device 21. In a preferred embodiment of the present invention, the anodizing device 21 is formed by overlapping plates.

**.** The anodizing device 21 comprises a base plate 22. The base plate 22 is flat shaped and comprises a set of guide pins 23 configured to engage the inside of dental implants. The base has fitting means 22a and gripping means 22b.

**.** Preferably, the base plate 22 is made of stainless steel, titanium alloys, polypropylene, PVC or other engineering polymer.

**.** The anodizing device 21 also comprises a housing plate 24. The housing plate comprises a set of holes 29, which can range from single to a 100x100 matrix, configured to align with the guide pins 23, with height variation between 1 mm and 50 mm from the base plate 22, which go through the entire housing plate 24.

**.** Housing plate 24 further comprises fitting means 24a, a recessed region with ramp 24c, and fastening means 24b.

**.** Preferably, housing plate 24 is made of stainless steel, titanium alloys, polypropylene, PVC or other engineering polymer.

**.** The anodizing device 21 also comprises an annular sealing structure 25. The sealing structure is configured to match the position of the guide pin assembly and the position of the holes 29, so that the perimeter of each hole 29 is sealed by the sealing structure 25. Furthermore, the sealing structure 25 can be a set of rings for each hole 29, or a single piece with the holes matching the holes 29.

**.** Preferably, the sealing structure is made of silicone, elastomers, fluorinated elastomers (Viton) or nitrile.

**.** Furthermore, the anodizing device 21 comprises a bottom plate 27. Preferably, the bottom plate 27 is solid and comprises fitting means 27a configured to match fitting means 24a of housing plate 24 and fastening means 27b configured to match the fastening means 24b of the housing plate 24.

**.** Preferably, the bottom plate 27 is made of stainless steel, titanium alloys, polypropylene, PVC or other engineering polymer.

**.** Anodizing device 21 also comprises guide pins 26 configured to align fitting means 22a, 24a, 27a. The guide pins have a wider band in order to establish a fixed distance between the housing plate 24 and the bottom plate 27.

**.** Furthermore, the anodizing device comprises locking elements 28 configured to fix and lock the housing plate 24 next to the bottom plate 27, through the fastening means 24b and 27b.

**.** Additionally, the locking element can be a bolt and nut system, spring, magnet, clamp, lever, butterfly or quick release clamp.

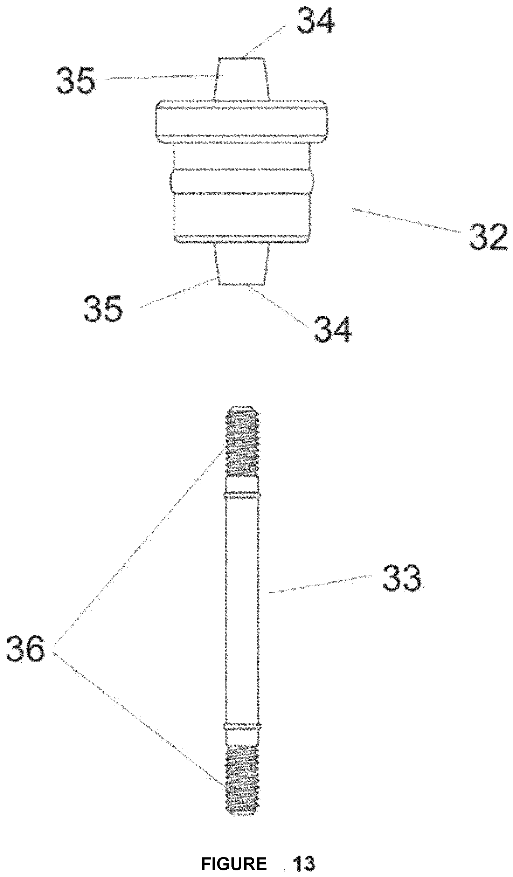

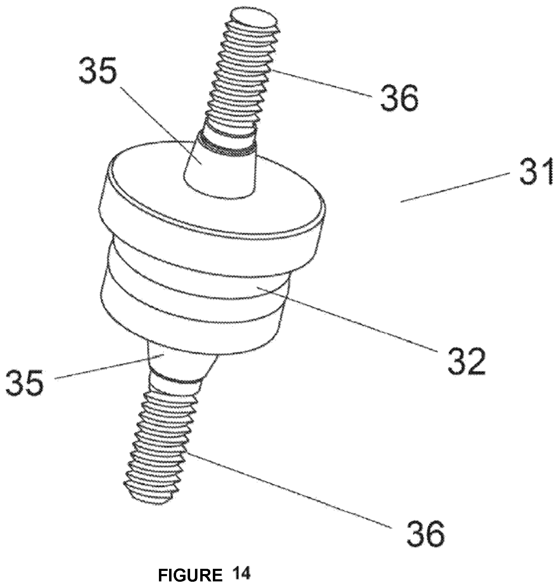

**.** Referring to Figures 13 and 14, the present invention comprises a chemical treatment and coating device 31. In a preferred embodiment of the present invention, the chemical treatment and coating device 31 comprises a sealing shaft 32 and an implant fixation pin 33.

**.** The sealing shaft 32 comprises a through hole 34 configured to receive said fixation pin 33. The through hole 34 is surrounded by sealing nozzles 35 at each end of the hole.

. The implant fixation pin 33 comprises two thread regions 36 configured to receive the internal thread of a dental implant, so that when the pin 33 passes through the sealing shaft 32, at least two implant fixation regions are created in that access to the internal surface of the implant is impossible, as illustrated in Figure 14.

. Preferably, the implant fixation pin 33 is made of a material among: titanium alloys, stainless steel or polymer.

. Preferably, the sealing shaft 32 is made of a material among: silicone, elastomers, fluorinated elastomers (Viton), nitrile.

. Furthermore, configurations are envisaged in which the sealing shaft is a tray with nozzle dies and a through hole, so that a plurality of implants can be threaded.

### Process description

. The implant manufacturing process takes place in three main steps: shearing and machining of the single body 11, anodizing the internal part of the implant through the anodizing device 21, acid etching and coating on the external part of the implant through the chemical treatment device 31.

. In a first machining step, the single body 11 is fixed to the guide bush 15 through the elongated region 14 and the clamp holder shaft 13. The stop 16 ensures that the relative distance of the single body 11 to the guide bush 15, measured in the direction of rotation shaft of the turning machine, is a constant and known distance, since the stop 16 touches the guide bush 15 and has a known size.

**.** With reference to Figure 1, the guide bush 15 is fixed to the turning machine through the guide bush 15 that guarantees the alignment of the part with the turning machine.

**.** After the fixation of the single body 11, there is a shear between the clamp holder shaft 13 and the dental implant body 12, so that the machining of the implant body 12 is continued, which is carried out until the threaded surface of the internal prosthetic connection of the implant is obtained.

**.** As an advantage, the single body 11 is perfectly aligned with the shaft and the reference point of the machine, increasing the final quality of the internal machined part of the implants.

**.** In a second anodizing step, the anodizing device 21 is configured to accommodate dental implants in order to isolate the inner surface from the outer surface of the implant.

**.** The assembly of the anodizing device 21 preferably takes place in an inverted manner.

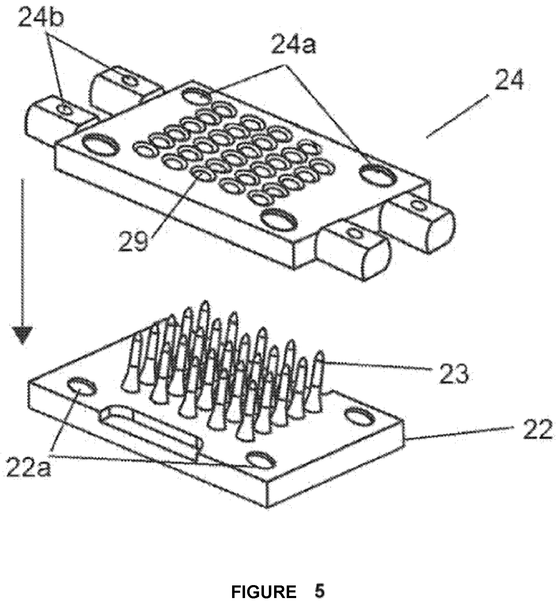

**.** With reference to Figure 5, the assembly of the device starts with the positioning of the base 22, so that the guide pins 23 face upwards.

**.** Housing plate 24 is placed on base 22 so that guide pins 23 pass through holes 29 in housing plate.

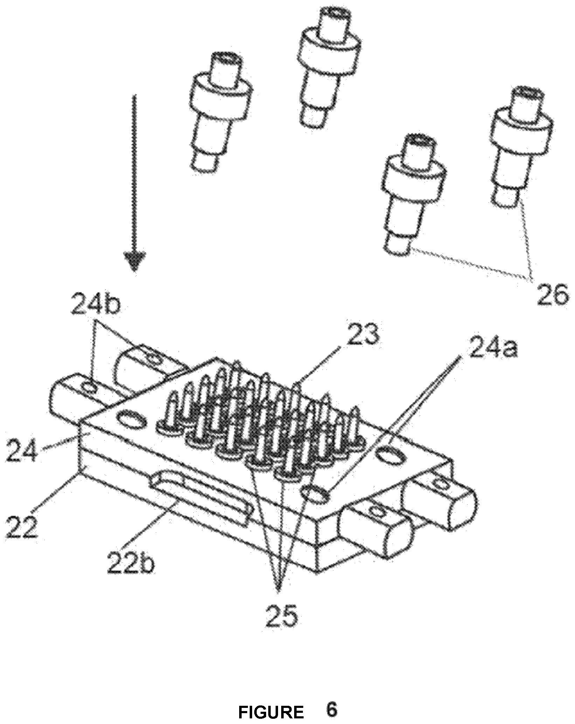

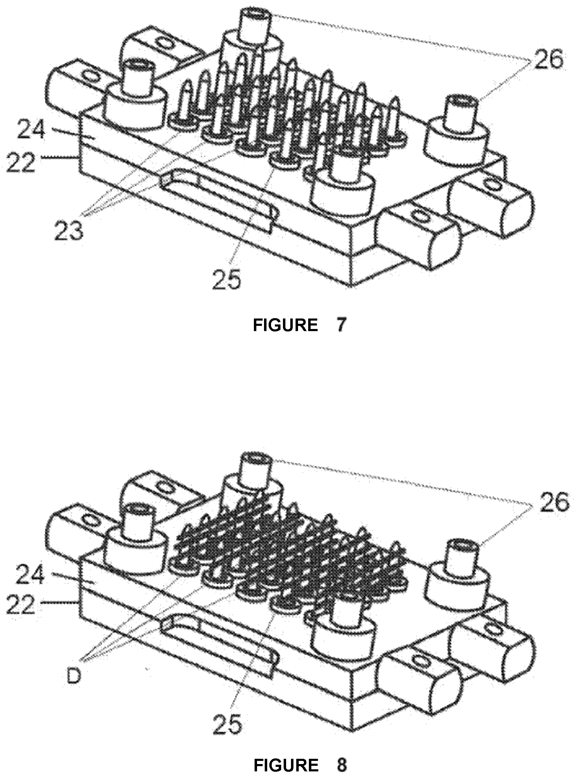

**.** With reference to Figures 6 and 7, the height guides 26 are inserted in the fitting means 22a and 24a, by interference, in order to align the plates 22 and 24. And the sealing structure 25 is placed under the housing plate of so that it is around the tip of the guide pins 23.

**.** Dental implants (D) are inserted into the device, so that the internal part of the implant fits over the tip of the guide pins 23, and its coronal end is in contact with the sealing structure 25, as illustrated in Figure 8.

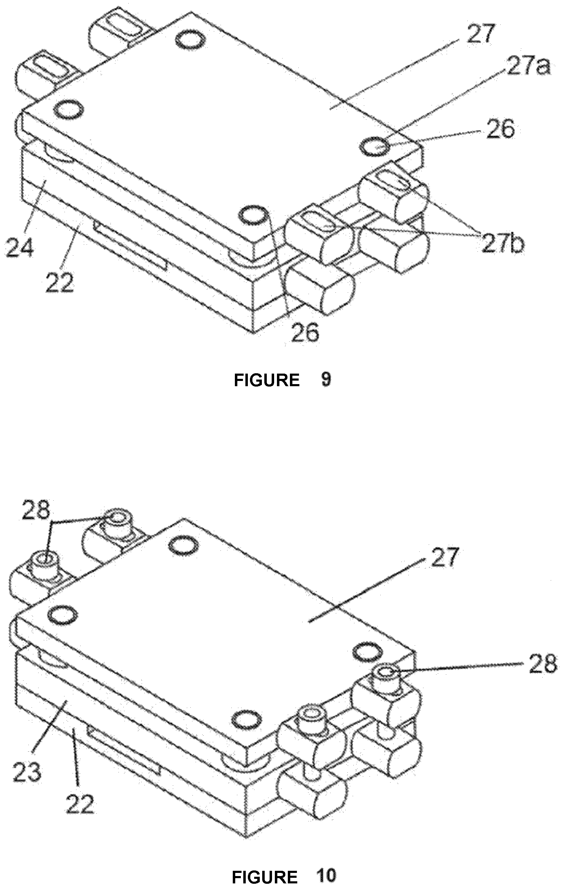

**.** Referring to Figure 9, the bottom plate 27 is supported under the device and aligned through engagement with the fitting means 27a and the height guides 26.

**.** The locking elements 28 are inserted by the fastening means 27b and 24b, and locked to join the housing plate 24 to the bottom plate 27. The locking element 28 forces the plates 24 and 27 so that the coronal end of the implant is sealed by the sealing structure 25, as illustrated in Figure 10.

**.** Referring to Figures 9 and 10, the anodizing device 21 is turned over, and the base 22 is removed, along with the guide pins 23.

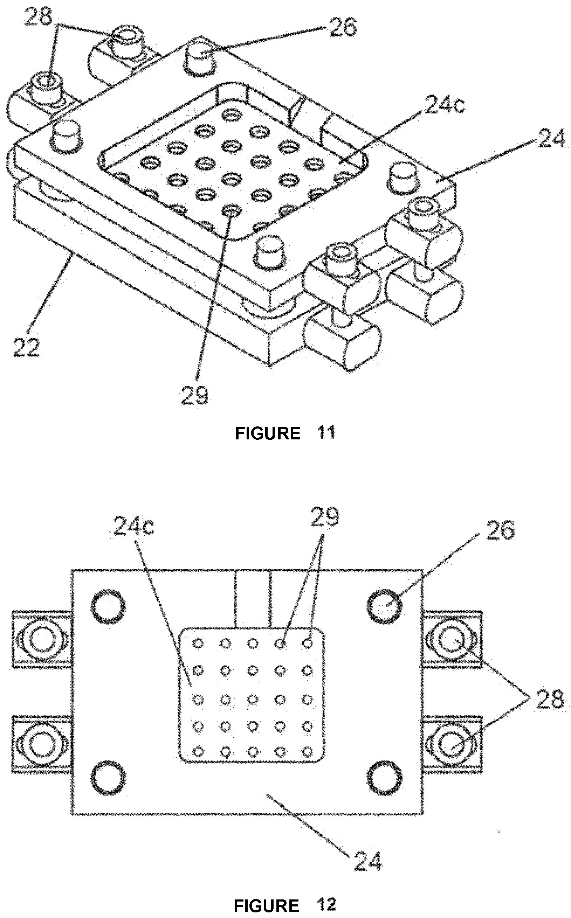

**.** With the removal of the base 22, illustrated by Figure 12, the device allows the treatment solution to be poured into the recessed region 24c and said solution to enter the holes 29 and come into contact with the internal region of the implant. The sealing structure 25, positioned between the housing plate 24 and the coronal end of the implant, prevents the solution from coming into contact with the outside of the implant. The sealing occurs because the housing plate 24 and the bottom plate 27 exert a compression force on the implants, due to the tensioned locking element 28.

**.** In this way, the anodizing device 21 is adapted to receive the treatment solution and guarantee the anodizing of the internal part of multiple implants at the same time. Furthermore, the anodizing device 21 can be easily incorporated into automatic production lines since it is enough to program the pouring of the solution over the recessed region 24c. The recessed region 24c also prevents the treatment solution from overflowing or leaking to the sides of the device, which would cause the solution to come into contact with external regions of the implant.

**.** In a third chemical treatment step, the chemical treatment and coating device 31 is configured to accommodate dental implants to isolate the outer surface from the inner surface of the implant.

**.** The assembly of the chemical treatment and coating device 31 takes place by passing the implant fixation pin 33 through the sealing shaft 32, so that each end of the chemical treatment and coating device 31 has a thread region 36.

**.** The thread region is configured to receive the internal thread of the internal region of the implant (D) machined and anodized. When the implants are fixed in both thread regions 36, the coronal ends of the implants make contact with the sealing nozzles 35, preventing a liquid solution from coming into contact with the internal part of the implants, allowing chemical treatments, such as acid etching and/or covering the implant with a layer of carbon nanostructures in the external region, preserving the characteristics obtained in the anodizing steps.

**.** In this way, the chemical treatment and coating device 31 is adapted to be submerged in a treatment solution and ensure the chemical treatment of the external part of multiple implants at the same time.

**.** Furthermore, the chemical treatment and coating device 31 is adapted to be submerged in a solution containing carbon nanostructures such as, preferably, graphene oxide, reduced graphene oxide, graphene, carbon nanotubes etc. and undergo an application of electric current for a determined time according to the number of implants and the type of coating desired, preserving the anodized internal part.

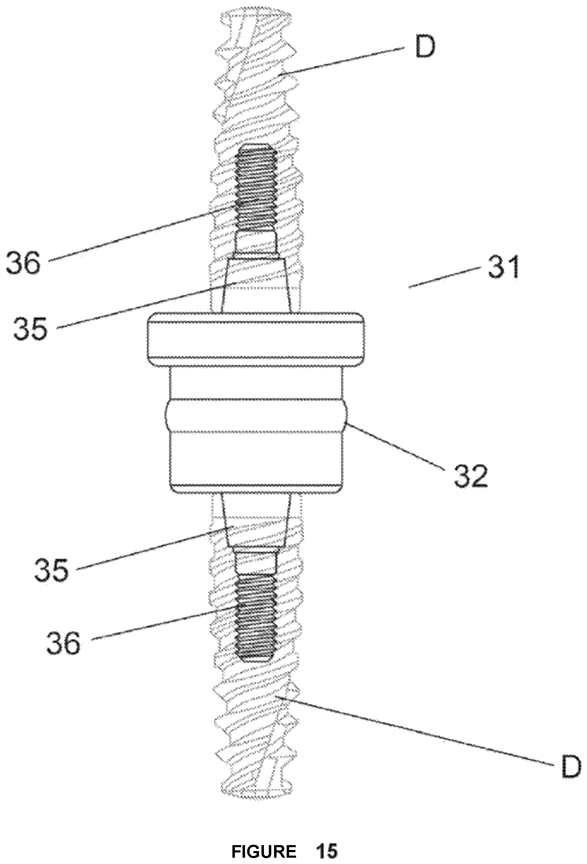

**.** Furthermore, the chemical treatment and coating device 31 can be easily incorporated into automatic production lines, since it is enough to program the device to be poured into a container with the desired solution, as shown in Figure 15.

. Having described some examples of preferred embodiments of the present invention, it should be understood that the scope of the present invention covers other possible variations of the described inventive concept, being limited only by the content of the claims only, including the possible equivalents.

## Claims

1. Device for anodizing a dental implant (21), **characterized by** comprising:
- a base plate (22);
- said base plate (22) comprising a set of guide pins (23) configured to engage in the internal part of dental implants;
- a housing plate (24);
- said housing plate comprising a set of holes (29) configured so as to align with the guide pins (23) of the base plate (22), said holes (29) traversing the entire housing plate (24);
- a sealing structure (25);
- said sealing structure (25) being configured to match the position of the set of guide pins (23) and the position of the holes (29);
- a bottom plate (27);
- a plurality of guide pins (26); configured to align the base (22), housing (24) and top (27) plates; and
- locking elements (28), configured so as to fix and lock the housing plate (24) next to the bottom plate (25);
wherein the base (22) has fitting means (22a) and gripping means (22b), and the housing plate (24) further comprising fitting means (24a), a recessed region with ramp (24c), and means fastening means (24b), and the bottom plate (27) comprises fitting means (27a) configured to match the fitting means (24a) of the housing plate (24) and fastening means (27b) configured to match the fastening means (24b) of the housing plate (24).

2. Device for anodizing a dental implant (21), according to claim 1, **characterized by** said device being assembled by positioning the base (22) so that the guide pins (23) face upwards and the housing plate ( 24) configured to fit over the base (22), so that the guide pins (23) pass through the holes (29) of the housing plate (24) and the height guides (26) are inserted by means of fitting of the base (22a) and the housing plate (24); and the sealing structure (25) configured to be placed over the housing plate (24) so that it is around the tip of the guide pins (23); and the bottom plate (27) is supported under the device and aligned through the engagement of fitting means (27a) with the height guides (26) and the locking elements (28) are inserted by the fastening means (27b) and (24b), and locked in order to join the housing plate (24) to the bottom plate (27), and the height guides (26) have a wider band in order to establish a fixed distance between the housing (24) and bottom plate (27).

3. Device for anodizing a dental implant (21), according to claims 1 and 2, **characterized by** said device being configured to receive multiple dental implants, the implants being inserted into the device (21), so that the internal part of the implant fits into the tip of the guide pins (23), and its coronal end is in contact with the sealing structure (25).

4. Device for anodizing a dental implant (21), according to claim 3, **characterized by** the locking element (28) applying a compression force between the housing (24) and bottom (27) plates, so that the structure seal (25) seals the meeting of the internal and external surface of the implant, at the coronal end of the implant.

5. Device for anodizing a dental implant (21), according to claims 1 to 4, **characterized by** after fixing the implant, the base (22) being removed, together with the guide pins (23) in order to allow a treatment solution being poured into the recessed region (4b) of the housing plate (24) and that said solution enters the holes (29) and comes into contact with the internal region of the implant.

6. Device for anodizing a dental implant (21) according to any one of claims 1 to 5, **characterized by** the base plate (22), the housing plate (24) and the bottom plate (27) being made of a resistant material among: stainless steel, titanium alloys, polypropylene, PVC or other engineering polymer.

7. Device for anodizing a dental implant (21), according to any one of claims 1 to 6, **characterized by** the sealing structure (25) being made of a material among: silicone, elastomers, fluorinated elastomers (Viton) or nitrile.

8. Device for anodizing a dental implant (21), according to any one of claims 1 to 7, **characterized by** the locking element (28) being one of the following types: screw and nut, spring, magnet, clamp, lever, butterfly or quick release clamp.

9. Device for chemical treatment and coating of a dental implant (31), **characterized by** comprising:
- a sealing shaft (32);
- the sealing shaft (32) comprising a through hole (34);
- the through hole (34) being surrounded by sealing nozzles (35) at each end of the hole (34);
- an implant fixation pin (33);
- the implant fixation pin (33) comprising two thread regions (36);
wherein said through hole (34) is configured to receive said fixation pin (33) and the two thread regions (36) are configured to receive the internal thread of a dental implant, so that when passing said fixation pin (33) by said sealing shaft (32), at least two implant fixation regions are created, so that the coronal region of the implant is in contact with the nozzles (35) and access to the internal surface of the implant it is impossible.

10. Device for chemical treatment and coating of a dental implant (31), according to claim 9, **characterized by** the sealing shaft (32) having the shape of a tray with nozzle matrices (35) and a through hole (34), in a that a plurality of implants can be threaded at each step.

11. Device for chemical treatment and coating of dental implant (31), according to any one of claims 9 and 10, **characterized by** the implant fixation pin (33) being made of a material among: titanium alloys, stainless steel or polymer.

12. Device for chemical treatment and coating of a dental implant (31), according to any one of claims 9 and 10, **characterized by** the sealing shaft (32) being made of a material among: silicone, elastomers, fluorinated elastomers (Viton), nitrile.

13. Single body (11) **characterized by** comprising:
- an implant body (12) or dental implant;
- a clamp holder shaft (13) made of metallic material fused to the end of the implant body (12), so as to form a single piece (11);
- the clamp holder shaft (13) further comprising an elongated region (14) configured to be fixed to a guide bush (15);
said clamp holder shaft (13) also comprising a stop (16) that delimits the depth of the elongated region (14) that will enter the guide bush (15) and the guide bush (15) keeps said implant (12) attached to a turning machine, and the stop (16) abuts the guide bush (15), so that the relative distance of the implant (12) to the guide bush (15), measured in the direction of the axis of rotation of the turning machine, is a constant distance.

14. Single body (11), according to claim 13, **characterized by** being made via additive manufacturing.

15. Single body (11), according to claim 14, **characterized by** being made of at least one material among: titanium alloys, nickel alloys, chromium alloys, aluminum alloys and stainless steel.

16. Single body machining process (11), as defined in claims 13 to 15, **characterized by** comprising the following steps:
- making a clamp holder shaft (13) next to a preformed dental implant body (12) by means of selective laser fusion in order to create a single body piece (11);
- attachment of the single body (11) to a guide bush (15) through the elongated region (14) of the clamp holder shaft (13);
- attachment of the single body (11) to a turning machine through the guide bush (15);
- shear between the implant body (12) and the clamp holder shaft (13);
- machining of the prosthetic connection of the internal surface of the implant body in order to obtain a 3D printed dental implant with the internal part machined; and
- perform the finishing of the surface of the implant.

17. Process for machining a single body (11), according to claim 16, **characterized by** being done automatically.

18. Dental implant anodizing process, using an anodizing device (21) as defined in claims 1 to 8, **characterized by** comprising the following steps:
- assembly of the anodizing device (21) with implant comprising:
- position the base (22) so that the guide pins (23) face upwards;
- attach the housing plate (24) so that the guide pins (23) pass through the holes (29) in the housing plate (24);
- inserting the height guides (26) by fitting means (24a) of the base (22) and the housing plate (24);
- place the sealing structure (25) on the housing plate (24) so that it is around the tip of the guide pins (23);
- inserting dental implants into the device (21), so that the internal part of the implant fits over the tip of the guide pins (23), and its coronal end is in contact with the sealing structure (25);
- coupling the bottom plate (27) under the device (21) and aligning said bottom plate (27) by engaging the fitting means (27a) with the height guides (26);
- insert locking elements (28) through the fastening means (27b) and (24b);
- lock the locking elements (28) so as to join the housing plate (24) to the bottom plate (27);
- removal of the base (22) together with the guide pins (23);
- pour an anodizing solution into the recessed region (24c) of the housing plate (24) so that said solution enters the holes (29) and comes into contact with the internal region of the implant;
- removing the locking elements (28) and separating the bottom plate (27) and the housing plate (24); and
- removal of the implants.

19. Dental implant anodizing process, according to claim 18, **characterized by** being done automatically.

20. Dental implant anodizing process, according to claims 18 and 19, **characterized by** being performed on a plurality of implants simultaneously.

21. Process for chemical treatment, using a device for chemical treatment and coating of a dental implant (31), as defined in claims 9 to 12, **characterized by** comprising the following steps:
- assembly of the chemical treatment device (31) comprising:
- passage of the implant fixation pin (33) through the sealing shaft (32), so that each end of the chemical treatment and coating device (31) has a thread region (36);
- thread of the internal thread of the implants in both thread regions (36), so that the coronal ends of the implants make contact with the sealing nozzles (35), preventing a liquid solution from coming into contact with the internal part of the implants;
- immersion of the device (31) with implant in a treatment solution for a sufficient time;
- removal the device (31) from the treatment solution;
- connection and immersion of the device (31) in a solution containing carbon nanostructures (graphene oxide, reduced graphene oxide, graphene, carbon nanotubes etc.);
- application of electric current for a determined time according to the number of implants and the type of coverage desired; and
- removal of the implants.

22. Process for chemical treatment, using a device for chemical treatment, according to claim 21, **characterized by** being done automatically.

23. Process for chemical treatment, using a device for chemical treatment, according to claims 21 and 22, **characterized by** being performed on a plurality of implants simultaneously.

24. Manufacturing process of a dental implant **characterized by** comprising the following steps:
- carry out a machining process for a single body (11) comprising the following steps:
- confection of a single body (11) through selective laser fusion of a clamp holder shaft (13) to a preformed implant body (12);
- attachment of the single body (11) to a guide bush (15) through the elongated region (14) of the clamp holder shaft (13);
- attachment of the single body (11) to a turning machine through the guide bush (15);
- shear between the implant body (12) and the clamp holder shaft (13);
- machining of the prosthetic connection of the internal surface of the implant body in order to obtain a 3D printed dental implant with the internal part machined; and
- finish the surface of the implant;
- carry out an anodizing process of the 3D printed dental implants, using an anodizing device (21) comprising the following steps:
- assembly of the anodizing device (21) with implant comprising:
- position the base (22) so that the guide pins (23) face upwards;
- attach the housing plate (24) so that the guide pins (23) pass through the holes (29) in the housing plate (24);
- insert the height guides (26) by fitting means (24a) of the base (22) and the housing plate (24);
- place the sealing structure (25) on the housing plate (24) so that it is around the tip of the guide pins (23);
- insert dental implants into the device (21), so that the internal part of the implant fits over the tip of the guide pins (23), and its coronal end is in contact with the sealing structure (25);
- couple the bottom plate (27) under the device (21) and aligning said bottom plate (27) by engaging the fitting means (27a) with the height guides (26);
- insert locking elements (28) through the fastening means (27b) and (24b); and
- lock the locking elements (28) so as to join the housing plate (24) to the bottom plate (27);
- removal of the base (22) together with the guide pins (23);
- pour an anodizing solution into the recessed region (24c) of the housing plate (24) so that said solution enters the holes (29) and comes into contact with the internal region of the implant;
- remove the locking elements (28) and separate the bottom plate (27) and the housing plate (24); and
- removal of implants;
- carry out a process for chemical treatment of 3D printed dental implants, using a device for chemical treatment and coating of dental implants (31) comprising the following steps:
- assembly of the chemical treatment and coating device (31) comprising:
- passage of the implant fixation pin (33) through the sealing shaft (32), so that each end of the chemical treatment device (31) has a thread region (36);
- thread of the internal thread of the implants in both thread regions (36), so that the coronal ends of the implants make contact with the sealing nozzles (35), preventing a liquid solution from coming into contact with the internal part of the implants;
- immersion of the device (31) with implant in a treatment solution for a sufficient time;
- remove the device (31) from the treatment solution;
- connection and immersion of the device (31) in a solution containing carbon nanostructures (graphene oxide, reduced graphene oxide, graphene, carbon nanotubes etc.);
- application of electric current for a determined time according to the number of implants and the type of coverage desired; and
- removal of the implants.

25. Manufacturing process of a dental implant, according to claim 24, **characterized by** being done automatically.

26. Manufacturing process of a dental implant, according to claim 25, **characterized by** being carried out for the manufacture of a plurality of implants simultaneously.

27. Dental implant, **characterized by** being made according to a manufacturing process as defined in claims 24 to 26.
